# EUROPEAN PATENT APPLICATION

(11) **EP 4 393 535 A1**
(43) Date of publication of application: **03.07.2024**
(21) Application number: 23218178.4
(22) Date of filing: 19.12.2023
(51) Int. Cl.: A61M 35/00

(54) **BRUSH/FOAM-BASED APPLICATOR FOR DISPENSING TISSUE ADHESIVE**

(30) Priority: 20.12.2022 US 202218084644
(71) Applicant: Becton, Dickinson and Company, Franklin Lakes, NJ 07417 (US)
(72) Inventor: KUMAR, Manish, 802302 Arrah (IN); NALAWADE, Praveen, 560054 Bangaluru (IN); PRASAD, Shishir, Ramsey, 07446 (US); SURYAVANSHI, Ajay, 411052 Pune (IN)
(74) Representative: dompatent von Kreisler Selting Werner - Partnerschaft von Patent- und Rechtsanwälten mbB

(57) **Abstract**

An adhesive applicator includes a body member defining an adhesive reservoir, a nozzle positioned within and extending from the body member, and an application member configured to receive the nozzle, wherein the application member is configured to receive an adhesive held in the adhesive reservoir upon pressure being applied to the body member.

## Description

### CROSS-REFERENCE TO RELATED APPLICATION

The present application claims priority to United States Utility Application No. 18/084,644 entitled "Brush/Foam-Based Applicator for Dispensing Tissue Adhesive" filed December 20, 2022, the entire disclosure of which is hereby incorporated by reference in its entirety.

### BACKGROUND OF THE INVENTION

### Field of the Invention

The present disclosure is generally directed to an applicator for dispensing tissue adhesive for a medical item and, more particularly, is directed to a brush- or foam-based applicator for dispensing tissue adhesive for securing a catheter line and/or catheter hub to a patient's skin surface.

### Description of Related Art

An intravenous (IV) set is currently used to access the circulatory system of a patient, enabling the administration of fluids and medications in a controlled, predictable manner. One particular example of an IV set is a catheter line and hub inserted into the patient. Typical IV sets may include a primary fluid flow line of tubular construction with one or more access points positioned along its length. Some of these access points can comprise access ports that allow for the administration of medications through either a syringe or by infusion through another IV set. A primary access point is located at one end of the IV set with a fluid source, such as saline, contrast medium, or blood, among other types of fluid. Secondary flow lines may be combined with the primary flow line to provide additional or alternative fluids to the patient.

Use of IV sets has now become integral at every stage of medical care, from the site of an accident or injury, through transport to the hospital, during emergency room and surgical procedures, and potentially continuing into the Intensive Care Unit and general hospital care. At each stage of procedure or treatment of the patient, different medical personnel typically become involved with attending to the patient.

When multiple medical personnel are involved, the combinations of procedures involving the multiple IV sets become even more complex and can lead to greater risk of the IV line becoming dislodged from the patient's vasculature. This is particularly true with respect to surgical procedures in an operating room in which medical personnel may be attending to several different issues or problems with a patient that require the medical personnel to move about the patient. In addition to the surgical staff having to focus on specific medical problems, other personnel may be involved, such as an anesthesia provider who typically administers anesthesia medications via the IV set, all of which increase the risk of an individual inadvertently dislodging the IV set or line from the patient.

After a patient has been attended to during a surgical procedure, the IV line and/or set may be kept in the patient's vasculature during recovery of the patient to provide needed nutrients and/or blood to the patient. In an effort to secure the IV line and/or hub to the patient's skin surface, a liquid adhesive may be applied to the patient's skin surface and/or the site of insertion of the IV line to secure the IV line and/or hub to the patient. The adhesive assists in reducing catheter movement, migration and dislodgement. There are other examples of skin adhesive which form a thin film on the patient's skin surface. Due to the volume of adhesive drops placed for IV catheter securement purposes, the curing time for the adhesive can be significantly long resulting in not having sufficient adhesion strength till curing is complete. This extended curing time leads to increase in procedure time or inadequate performance while the adhesive is being cured.

Therefore, there is a current need in the art for an applicator that dispenses adhesive which leads to reduced curing time with sufficient adhesion strength, while retaining the flexibility to improve overall procedure time. There is a further need for an applicator that can apply adhesive to areas which are difficult to reach due to an interfering distal end of an applicator nose, which leads to improper spread of adhesive on the patient's skin surface and/or the IV set/line.

### SUMMARY OF THE INVENTION

In view of the foregoing needs, an applicator according to one non-limiting embodiment or aspect of the present disclosure is provided. In one non-limiting embodiment or aspect of the present disclosure, an adhesive applicator may include a body member defining an adhesive reservoir, a nozzle positioned within and extending from the body member, and an application member configured to receive the nozzle, wherein the application member is configured to receive an adhesive held in the adhesive reservoir upon pressure being applied to the body member.

In one non-limiting embodiment or aspect of the present disclosure, the application member may be made of a foam material, a plurality of bristles, or a foam material and a plurality of bristles. The adhesive may be a tissue adhesive used for securing a catheter line and/or catheter hub to a patient's skin surface. The nozzle may be fixed within the body member. The nozzle may be free floating within the body member such that the nozzle is retractable into the body member. The nozzle may define a channel to direct the adhesive from the adhesive reservoir to a patient's skin surface. The body member may be formed as a sachet. The application member may have a wedged shape. An end cap may be removably attached to the body member to cover the application member and nozzle when the adhesive applicator is not being used. The end cap may include a central pin used to plug a channel defined in the nozzle when the end cap is held on the body member. The application member may be retractably positioned on the nozzle. A gripping member may be operatively connected to the application member to move the application member relative to the nozzle. The adhesive applicator may be configured to deliver at least one droplet of the adhesive to a patient's skin surface and to spread a thin film of the adhesive on the patient's skin surface using the application member.

In one non-limiting embodiment or aspect of the present disclosure, an adhesive applicator may include a body member defining an adhesive reservoir, an adhesive capsule held in the adhesive reservoir, a nozzle positioned within and extending from the body member, and an application member configured to receive the nozzle, wherein the application member is configured to receive an adhesive held in the adhesive reservoir upon pressure being applied to the body member.

In one non-limiting embodiment or aspect of the present disclosure, the adhesive capsule may be configured to break upon a pressure being applied to the body member. The adhesive capsule may be made of glass. A filter may be positioned in the nozzle. The application member may be made of a foam material, a plurality of bristles, or a foam material and a plurality of bristles.

In one non-limiting embodiment or aspect of the present disclosure, a method of applying an adhesive film to a patient's skin surface may include applying pressure to a body member of an adhesive applicator to direct an adhesive from an adhesive reservoir to a nozzle, directing the adhesive in a form of droplets from the nozzle to the patient's skin surface, and spreading the droplets of the adhesive into a thin film on the patient's skin surface using an application member of the adhesive applicator. The method may also include retracting the nozzle into the body member when the application member is pressed against the patient's skin surface to spread the droplets of the adhesive.

In accordance with an embodiment of the present invention, an adhesive applicator, includes a body member defining an adhesive reservoir; a nozzle positioned within and extending from the body member; and an application member configured to receive the nozzle, wherein the application member is configured to receive an adhesive held in the adhesive reservoir upon pressure being applied to the body member.

In accordance with an embodiment of the present invention, the application member is made of a foam material, a plurality of bristles, or a foam material and a plurality of bristles.

In accordance with an embodiment of the present invention, the adhesive is a tissue adhesive used for securing a catheter line and/or catheter hub to a patient's skin surface.

In accordance with an embodiment of the present invention, the nozzle is fixed within the body member.

In accordance with an embodiment of the present invention, the nozzle is free floating within the body member such that the nozzle is retractable into the body member.

In accordance with an embodiment of the present invention, the nozzle defines a channel to direct the adhesive from the adhesive reservoir to a patient's skin surface.

In accordance with an embodiment of the present invention, the body member is formed as a sachet.

In accordance with an embodiment of the present invention, the application member has a wedged shape.

In accordance with an embodiment of the present invention, further including an end cap removably attached to the body member to cover the application member and nozzle when the adhesive applicator is not being used.

In accordance with an embodiment of the present invention, the end cap includes a central pin used to plug a channel defined in the nozzle when the end cap is held on the body member.

In accordance with an embodiment of the present invention, the application member is retractably positioned on the nozzle.

In accordance with an embodiment of the present invention, the applicator further includes a gripping member operatively connected to the application member to move the application member relative to the nozzle.

In accordance with an embodiment of the present invention, the adhesive applicator is configured to deliver at least one droplet of the adhesive to a patient's skin surface and to spread a thin film of the adhesive on the patient's skin surface using the application member.

In accordance with an embodiment of the present invention, an adhesive applicator includes a body member defining an adhesive reservoir; an adhesive capsule held in the adhesive reservoir; a nozzle positioned within and extending from the body member; and an application member configured to receive the nozzle, wherein the application member is configured to receive an adhesive held in the adhesive reservoir upon pressure being applied to the body member.

In accordance with an embodiment of the present invention, the adhesive capsule is configured to break open to release adhesive upon a pressure being applied to the body member.

In accordance with an embodiment of the present invention, the adhesive capsule is made of glass.

In accordance with an embodiment of the present invention, the applicator further comprising a filter positioned in the nozzle to filter broken glass pieces after breaking glass capsule.

In accordance with an embodiment of the present invention, the application member is made of a foam material, a plurality of bristles, or a foam material and a plurality of bristles.

In accordance with an embodiment of the present invention, a method of applying an adhesive film to a patient's skin surface includes applying pressure to a body member of an adhesive applicator to direct an adhesive from an adhesive reservoir to a nozzle; directing the adhesive in a form of droplets from the nozzle to the patient's skin surface; and spreading the droplets of the adhesive into a thin film on the patient's skin surface using an application member of the adhesive applicator.

In accordance with an embodiment of the present invention, the method further includes retracting the nozzle into the body member when the application member is pressed against the patient's skin surface to spread the droplets of the adhesive.

### BRIEF DESCRIPTION OF THE DRAWINGS

Fig. 1 is a side view of an adhesive applicator according to one non-limiting embodiment or aspect of the present disclosure;
Fig. 2 is a cross-sectional view of the adhesive applicator of Fig. 1;
Fig. 3 is an exploded view of the adhesive applicator of Fig. 1;
Fig. 4 is a perspective view of the adhesive applicator of Fig. 1 applying a droplet of adhesive to a patient's skin surface;
Fig. 5 is a perspective view of the adhesive applicator of Fig. 1 spreading the droplet of adhesive on the patient's skin surface;
Fig. 6 is a side view of an adhesive applicator according to one non-limiting embodiment or aspect of the present disclosure;
Fig. 7 is a side view of an extended nozzle of the adhesive applicator of Fig. 6;
Fig. 8 is a side view of a retracted nozzle of the adhesive applicator of Fig. 6;
Fig. 9 is a side view of an adhesive applicator according to one non-limiting embodiment or aspect of the present disclosure;
Fig. 10 is a side view of an exposed nozzle of the adhesive applicator of Fig. 9;
Fig. 11 is a side view of a retracted nozzle of the adhesive applicator of Fig. 9;
Fig. 12 is a side view of an adhesive applicator according to one non-limiting embodiment or aspect of the present disclosure;
Fig. 13 is a side view of the adhesive applicator of Fig. 12 in an un-squeezed position; and
Fig. 14 is a side view of the adhesive applicator of Fig. 12 in a squeezed position.

### DESCRIPTION OF THE DISCLOSURE

The following description is provided to enable those skilled in the art to make and use the described embodiments contemplated for carrying out the invention. Various modifications, equivalents, variations, and alternatives, however, will remain readily apparent to those skilled in the art. Any and all such modifications, variations, equivalents, and alternatives are intended to fall within the spirit and scope of the present invention.

For purposes of the description hereinafter, the terms "upper", "lower", "right", "left", "vertical", "horizontal", "top", "bottom", "lateral", "longitudinal", and derivatives thereof shall relate to the invention as it is oriented in the drawing figures. However, it is to be understood that the invention may assume alternative variations and step sequences, except where expressly specified to the contrary. It is also to be understood that the specific devices and processes illustrated in the attached drawings, and described in the following specification, are simply exemplary embodiments of the invention. Hence, specific dimensions and other physical characteristics related to the embodiments disclosed herein are not to be considered as limiting.

The present disclosure is generally directed to an applicator for dispensing tissue adhesive for a medical item and, more particularly, is directed to a brush- or foam-based applicator for dispensing tissue adhesive for securing a catheter line and/or catheter hub to a patient's skin surface.

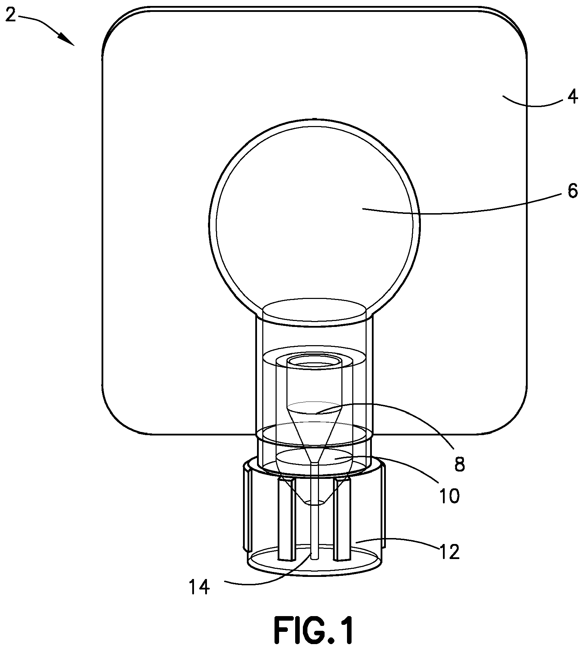

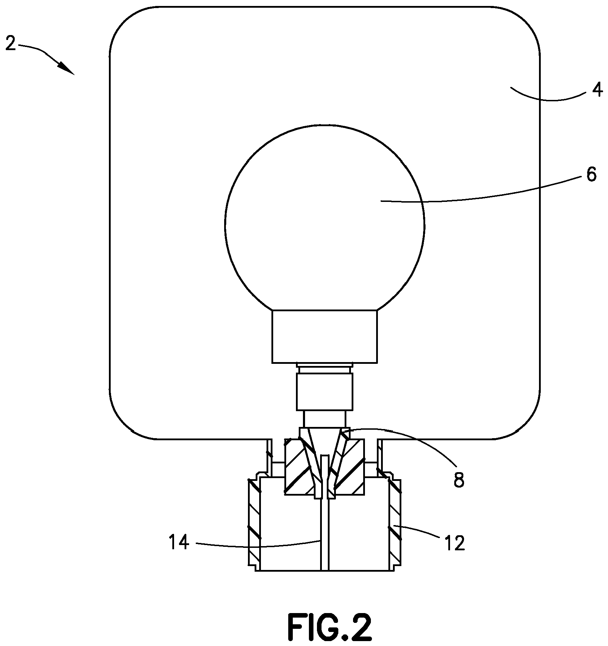

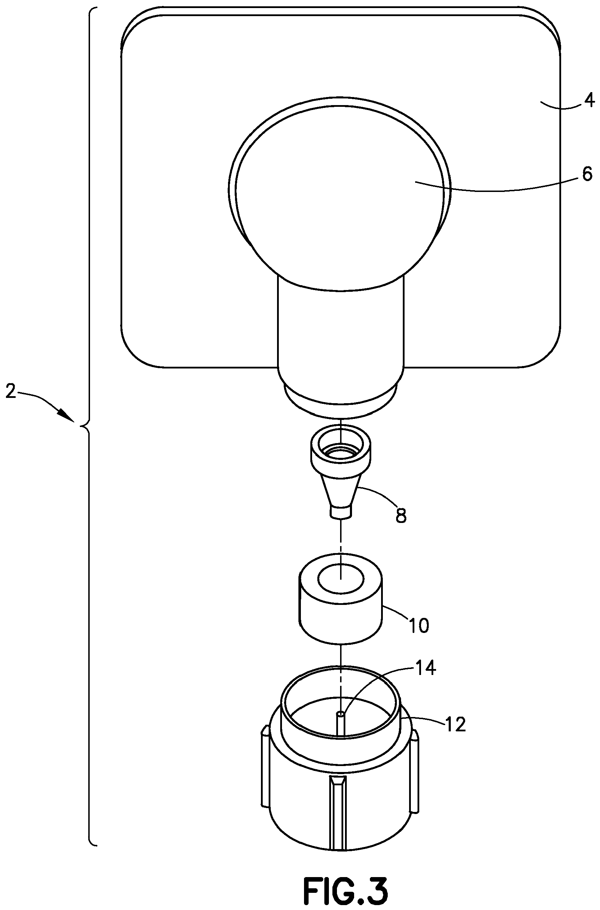

With reference to Figs. 1-3, according to one non-limiting embodiment or aspect of the present disclosure, an adhesive applicator 2 is shown and described in detail. The adhesive applicator 2 may be used to apply an adhesive, such as a tissue adhesive, to a patient's skin surface to assist in securing a catheter line and/or catheter hub to the patient's skin surface or site of insertion for the catheter line. The adhesive applicator 2 may include a body member 4 and an adhesive reservoir 6 defined in the body member 4. In one example, the body member 4 may be formed as a square-shaped body member, but it is also contemplated that alternative shapes and/or sizes for the body member 4 are considered. In one example, the body member 4 is formed as a sachet. The body member 4 may be formed with an internal metal foil-based lining to store the tissue adhesive in the adhesive reservoir 6 and to provide a barrier to the environment external to the adhesive applicator 2.

In one non-limiting embodiment or aspect of the present disclosure, the tissue adhesive may be filled in the adhesive reservoir 6 and may be released from the adhesive reservoir 6 by squeezing or applying pressure to the body member 4. Upon squeezing or applying pressure to the body member 4, the tissue adhesive is released from the adhesive reservoir 6 and is directed to a nozzle 8 positioned beneath and in fluid communication with the adhesive reservoir 6. In one example, the adhesive reservoir 6 may be formed as a thermoformed bulb-like structure. In one non-limiting embodiment or aspect of the present disclosure, the nozzle 8 may be fixed to the body member 4 or adhesive reservoir 6 or may be floating in the cavity defined by the body member 4.

With reference to Figs. 1-3, according to one non-limiting embodiment or aspect of the present disclosure, the adhesive applicator 2 may also include an application member 10 configured to receive the tissue adhesive from the nozzle 8 after or while the body member 4 is squeezed. In one example, the application member 10 may be made of foam. In another example, the application member 10 may include a plurality of bristles. In another example, the application member 10 may include foam and a plurality of bristles. The application member 10 may be operatively connected to a distal end of the body member 4 and may be configured to receive the nozzle 8 such that the nozzle 8 directs the tissue adhesive from the adhesive reservoir 6 to the application member 10. The tissue adhesive may be soaked into the application member 10 by squeezing the body member 4 to force the tissue adhesive from the adhesive reservoir 6 to the nozzle 8 and into the application member 10. The application member 10 may be configured to apply a thin layer of the tissue adhesive around the site of insertion for the catheter line to secure the catheter line in the site of insertion and secure the catheter hub to the patient's skin surface.

In the example of the application member 10 being made of foam, the application member 10 may be formed with a distinct pointed end with an opening defined therein to allow the tissue adhesive to flow from the nozzle 8 and through the opening of the application member 10 in the form of small droplets to direct the tissue adhesive to areas on the patient that may otherwise be difficult to reach, such as the site of insertion of the catheter line. Furthermore, upon dispensing the tissue adhesive droplets at the desired site on the patient, the tissue adhesive can be smeared on the site with the foam application member 10 to spread the tissue adhesive over a large area on the patient resulting in a thin layer of tissue of adhesive on the patient's skin surface, resulting in a faster curing time for the tissue adhesive for fixing and stabilizing the catheter line and/or catheter hub on the patient.

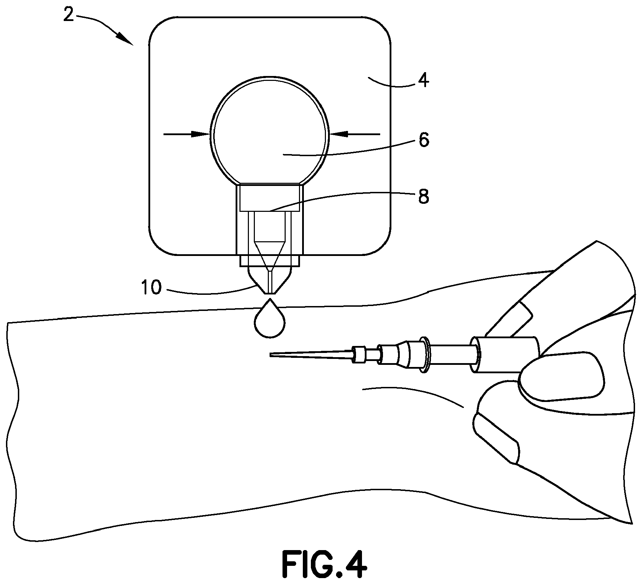

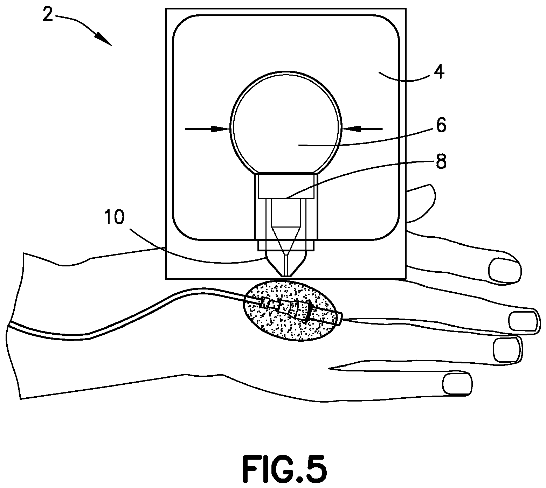

As shown in Figs. 4 and 5, in the example of the application member 10 being made of a foam and bristles, the design of the application member 10 allows the application member 10 to perform a dual function of dispensing droplets of the tissue adhesive through a central opening in the foam, as well as spreading the tissue adhesive on the patient's skin surface using the bristles, similar to a paint brush. The bristles of the application member 10 may also allow a user to apply the tissue adhesive underneath a catheter line or catheter hub, which is typically a difficult area to reach to apply tissue adhesive. By spreading the tissue adhesive with the bristles of the application member 10, the tissue adhesive can dry more quickly to decrease the amount of time needed to secure the catheter line and/or catheter hub to the patient.

With reference to Figs. 1-3, according to one non-limiting embodiment or aspect of the present disclosure, the adhesive applicator 2 may also include an end cap 12 to seal the internal components of the adhesive applicator 2 from the external environment to maintain the sterility of the tissue adhesive and the internal components of the adhesive applicator 2 during its shelf life, as well as to prevent exposure to air which may neutralize the effectiveness of the adhesive. The end cap 12 may be threadedly secured to the body member 4, but it is also contemplated that other securement means may be used to secure the end cap 12 to the body member 4. In one example, a central pin 14 may be operatively connected to an inner surface of the end cap 12. The central pin 14 may be configured to plug the opening of the nozzle 8 when the end cap 12 is held on the body member 4. The central pin 14 is configured to prevent the tissue adhesive to flow out of the nozzle 8 until the end cap 12 is removed before use of the adhesive applicator 2. In one non-limiting embodiment or aspect of the present disclosure, the adhesive applicator 2 may be sterilized using a single stage irradiation or e-beam method.

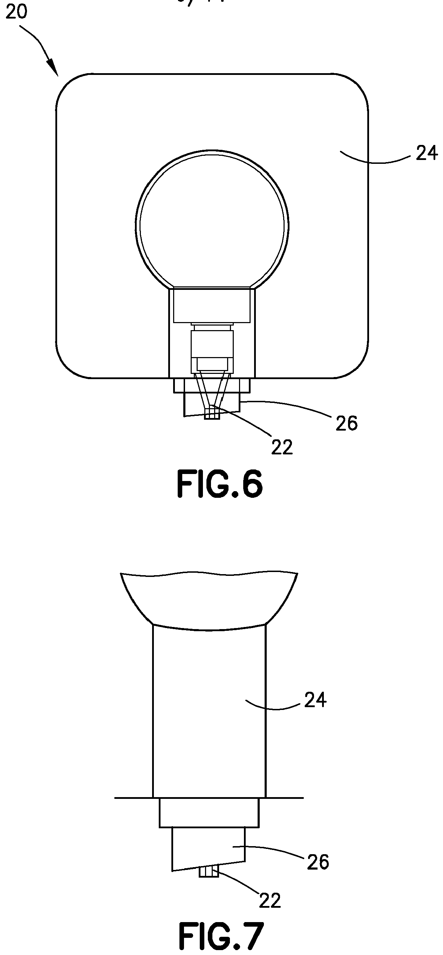

With reference to Figs. 6-8, according to another non-limiting embodiment or aspect of the present disclosure, an adhesive applicator 20 with a free-floating nozzle 22 is shown and described in detail. The free-floating nozzle 22 may be held in the body member 24 so as to be freely retractable within the body member 24 as the free-floating nozzle 22 is pressed against a patient's skin surface. As shown in Fig. 7, in one example, when the adhesive applicator 20 is not being pressed against the patient's skin surface, a distal end of the free-floating nozzle 22 extends distally past a distal surface of an application member 26. With the free-floating nozzle 22 extending out of the application member 26, the body member 24 can be squeezed to force tissue adhesive out of the free-floating nozzle 22 in droplets to be applied to the patient's skin surface. With the extended, un-retracted free-floating nozzle 22, the droplets of the tissue adhesive can be more accurately deposited near the site of insertion of a catheter line in the patient's skin surface. As the free-floating nozzle 22 is pressed against the patient's skin surface or a pressure is applied to the distal end of the free-floating nozzle 22, the free-floating nozzle 22 will retract into the body member 24 to level the patient's skin surface with the application member 26 to apply and spread the tissue adhesive on the patient's skin surface. By allowing the free-floating nozzle 22 to retract into the body member 24, a user can ergonomically spread the tissue adhesive droplets on the patient's skin surface to form a thin film of tissue adhesive using the foam and bristles of the application member 26 for quick drying of the tissue adhesive and securement of the catheter line and catheter hub. In one example, the application member 26 may have a wedged or tapered shape similar to a paintbrush or pen to spread the tissue adhesive on the patient's skin surface.

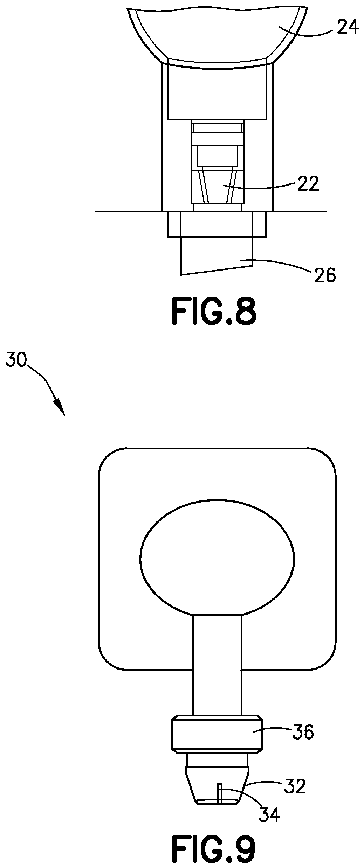

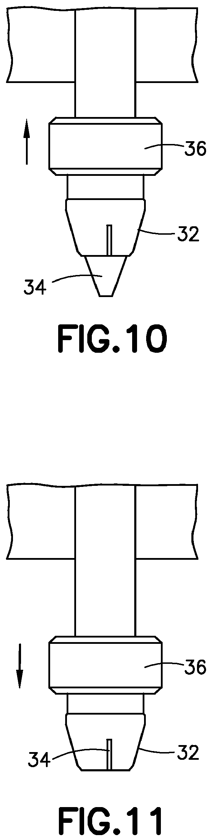

With reference to Figs. 9-11, according another non-limiting embodiment or aspect of the present disclosure, an adhesive applicator 30 with an adjustable application member 32 is shown and described in detail. The adjustable application member 32 may be held on a nozzle 34 of the adhesive applicator 30 so as to be freely retractable with respect to the nozzle 34. As shown in Fig. 9, in one example, a gripping member 36 may be operatively connected to the adjustable application member 32 to move the adjustable application member 32 relative to the nozzle 34 in an axial direction. In a first position, the adjustable application member 32 may be held at a proximal positon relative to the nozzle 34 to permit the nozzle to apply droplets of tissue adhesive to a patient's skin surface. In a second position, the adjustable application member 32 may be moved by the gripping member 36 to a distal position relative to the nozzle 34 so that the adjustable application member 32 can be used to spread the tissue adhesive on the patient's skin surface. In one example, the adjustable application member 32 may also include a locking member (not shown) to lock the adjustable application member 32 in a desired position. The gripping member 36 may be a circular member that surrounds the adjustable application member 32. The gripping member 36 may include a gripping material that assists in a user gripping and holding the gripping member 36 as the gripping member 36 is used to adjust the position of the adjustable application member 32. A user can ergonomically spread the tissue adhesive droplets on the patient's skin surface to form a thin film of tissue adhesive using the foam and bristles of the adjustable application member 32 for quick drying of the tissue adhesive and securement of the catheter line and catheter hub.

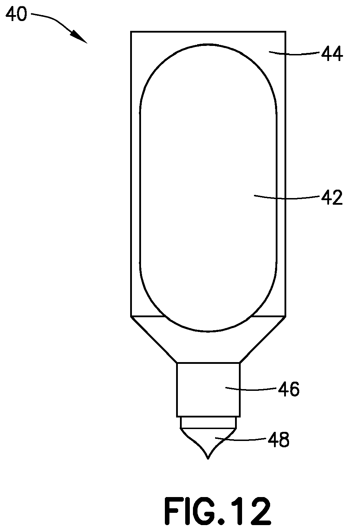

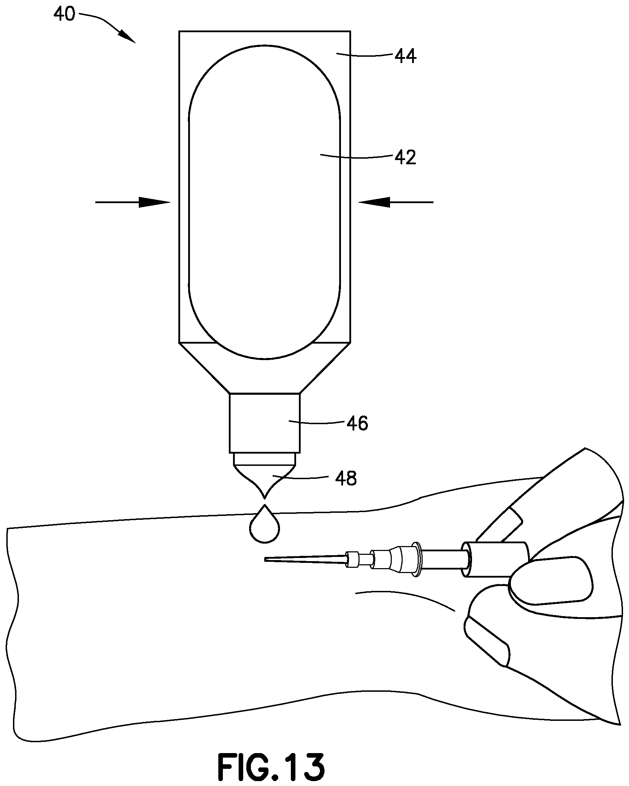

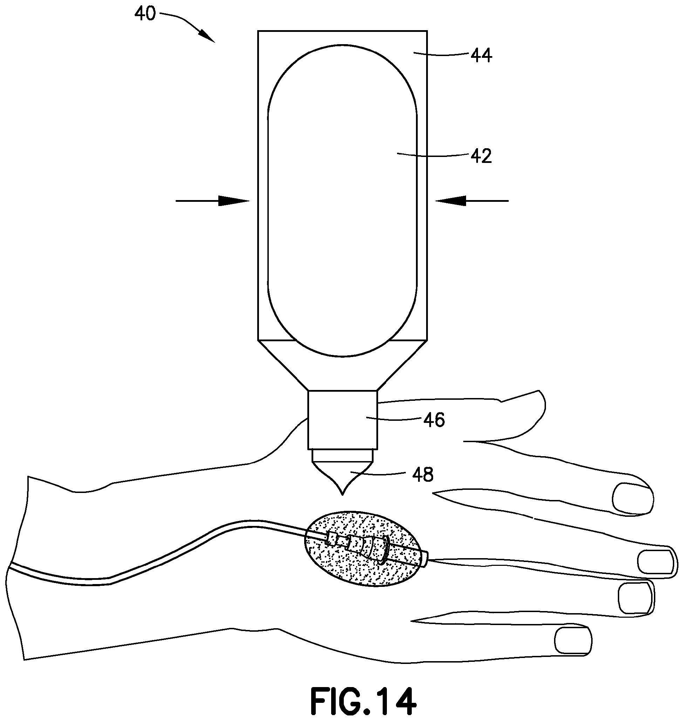

With reference to Figs. 12-14, according to another non-limiting embodiment or aspect of the present disclosure, an adhesive applicator 40 with an adhesive capsule 42 is shown and described in detail. The adhesive applicator 40 may have a body member 44 that defines a hollow cavity to receive the adhesive capsule 42. In one example, the adhesive capsule 42 may be made of glass and configured to hold a tissue adhesive. The adhesive capsule 42 may be broken to release the tissue adhesive for application to a patient's skin surface. As shown in Fig. 13, the body member 44 may be squeezed by the user to break the adhesive capsule 42, thereby releasing the tissue adhesive.

In one example of the present disclosure, a distal end of the body member 44 may have a filter 46 to allow the tissue adhesive to pass through while removing any glass shards or pieces from the adhesive capsule 42 out of the tissue adhesive to ensure no portions of the broken adhesive capsule 42 are placed on the patient's skin surface. In one example, positioned distally from the filter 46, an application member 48 may be operatively connected with or formed on the distal end of the body member 44. In certain configurations, the filter may be made of a cellulose-based paper, a non-woven polymer, such as, but not limited to polyester, polypropylene, polystyrene, and/or fiber glass. The application member 48 may be formed of a foam material, a plurality of bristles, or a foam material and a plurality of bristles. The application member 48 may be configured to apply droplets of the tissue adhesive on the patient's skin surface, as well spread the droplets of the tissue adhesive into a thin film on the patient's skin surface to reduce curing time and increase the amount of time needed to secure the catheter line or catheter hub to the patient's skin surface.

While embodiments of an adhesive applicator are shown in the accompanying figures and described hereinabove in detail, other embodiments will be apparent to, and readily made by, those skilled in the art without departing from the scope and spirit of the invention. Accordingly, the foregoing description is intended to be illustrative rather than restrictive. The invention described hereinabove is defined by the appended claims and all changes to the invention that fall within the meaning and the range of equivalency of the claims are to be embraced within their scope.

## Claims

1. An adhesive applicator, comprising:
a body member defining an adhesive reservoir;
a nozzle positioned within and extending from the body member; and
an application member configured to receive the nozzle, wherein the application member is configured to receive an adhesive held in the adhesive reservoir upon pressure being applied to the body member.

2. The adhesive applicator of claim 1, wherein the application member is made of a foam material, a plurality of bristles, or a foam material and a plurality of bristles.

3. The adhesive applicator of claim 1, the adhesive is a tissue adhesive used for securing a catheter line and/or catheter hub to a patient's skin surface.

4. The adhesive applicator of claim 1, wherein the nozzle is fixed within the body member.

5. The adhesive applicator of claim 1, wherein the nozzle is free floating within the body member such that the nozzle is retractable into the body member.

6. The adhesive applicator of claim 1, wherein the nozzle defines a channel to direct the adhesive from the adhesive reservoir to a patient's skin surface.

7. The adhesive applicator of claim 1, wherein the body member is formed as a sachet.

8. The adhesive applicator of claim 1, wherein the application member has a wedged shape.

9. The adhesive applicator of claim 1, further comprising an end cap removably attached to the body member to cover the application member and nozzle when the adhesive applicator is not being used.

10. The adhesive applicator of claim 9, wherein the end cap comprises a central pin used to plug a channel defined in the nozzle when the end cap is held on the body member.

11. The adhesive applicator of claim 1, wherein the application member is retractably positioned on the nozzle.

12. The adhesive applicator of claim 11, further comprising a gripping member operatively connected to the application member to move the application member relative to the nozzle.

13. The adhesive applicator of claim 1, wherein the adhesive applicator is configured to deliver at least one droplet of the adhesive to a patient's skin surface and to spread a thin film of the adhesive on the patient's skin surface using the application member.

14. An adhesive applicator, comprising:
a body member defining an adhesive reservoir;
an adhesive capsule held in the adhesive reservoir;
a nozzle positioned within and extending from the body member; and
an application member configured to receive the nozzle, wherein the application member is configured to receive an adhesive held in the adhesive reservoir upon pressure being applied to the body member.

15. The adhesive applicator of claim 14, wherein the adhesive capsule is configured to break open upon a pressure being applied to the body member.

16. The adhesive applicator of claim 14, wherein the adhesive capsule is made of glass.

17. The adhesive applicator of claim 14, further comprising a filter positioned in the nozzle.

18. The adhesive applicator of claim 14, wherein the application member is made of a foam material, a plurality of bristles, or a foam material and a plurality of bristles.

19. A method of applying an adhesive film to a patient's skin surface, the method comprising:
applying pressure to a body member of an adhesive applicator to direct an adhesive from an adhesive reservoir to a nozzle;
directing the adhesive in a form of droplets from the nozzle to the patient's skin surface; and
spreading the droplets of the adhesive into a thin film on the patient's skin surface using an application member of the adhesive applicator.

20. The method of claim 19, further comprising retracting the nozzle into the body member when the application member is pressed against the patient's skin surface to spread the droplets of the adhesive.
